# EUROPEAN PATENT SPECIFICATION

(11) **EP 2 650 261 B1**
(45) Date of publication and mention of the grant of the patent: **03.06.2015**
(21) Application number: 12163549.4
(22) Date of filing: 10.04.2012
(51) Int. Cl.: C02F 1/36, C02F 1/78, C02F 9/00, A61L 2/025, A61L 2/20, B67D 3/00

(54) **Disinfection device for water dispenser**
Desinfektionsvorrichtung für einen Wasserspender
Dispositif de désinfection pour distributeur d'eau

(43) Date of publication of application: 16.10.2013
(73) Proprietor: Scandinavian Innovation Group Oy, 29630 Pomarkku (FI)
(72) Inventor: Pawlow, Andrzej, LV-1005 Riga (LV)
(74) Representative: Anohins, Vladimirs

(56) References cited:
- US-A- 5 683 576
- US-A1- 2003 000 966
- US-A1- 2008 022 694
- US-B1- 6 312 608

## Description

### Technical Field

The present invention relates to disinfection devices for water dispensers, and more particularly, to disinfection devices for water dispensers having accumulating reservoir for drinking water filled from an external source. The device can be applied in new water dispensers or used in modernization of existing dispensers.

### Background Art

Many types of water dispensers for drinking water contain one or more inner accumulating reservoirs wherefrom drinking water is dispensed to the consumers. Designs of water dispensers are known wherein water from an external source, such as water main, filter or bottle, is accumulated in one or more inner reservoirs; for example, water may be accumulated and cooled within a cold-water reservoir, accumulated and carbonated within an aerated water reservoir, and accumulated in yet another reservoir for room temperature water. Dispensers of such type face the problem that they are not closed sterile systems and therefore during their operation various microorganisms may proliferate inside, also pathogenic, which may finally lead to deterioration of water quality, in particular to worse water taste or to proliferation of infectious diseases.

Therefore such dispensers need regular cleaning and sanitary treatment which is a lasting and labour consuming operation. Besides, during regular cleaning the dispenser is not available for operation. Therefore there is a need for a device for maintaining a dispenser clean in the process of operation.

Systems are known where ozone is delivered into water containing in the accumulating reservoir in order to disinfect it. For example, US patent 6085540 proposes switching on the ozone generator for certain time intervals set by timer in order to feed ozone into water containing in the accumulating reservoir. US patent 5683576 also suggests treating water in the dispenser by ozone as well as increasing ozone solubility in water through the use of ultrasonic device placed within the water reservoir. In US patent application 2003/0000966 ozone is proposed to be simultaneously delivered into the water reservoir, into the tap and into the pipeline connecting the water reservoir and tap.

However, abovementioned and other known systems using ozone have the following inherent drawbacks. First, high enough ozone concentration is required which seriously affects taste properties of water and needs much time to remove ozone from the water, that is, after beginning and throughout the treatment period that could be up to several hours the dispenser is not available to consumers.

Second, ozone treatment is not sufficient for cleaning from biofilm forming on water-contacting walls in hard-to-reach places, particularly in a water channel connecting the accumulating reservoir for drinking water and the water dispensing device or tap; besides, even deactivated biofilm is a favourable ambience for subsequent development of biological pollution.

We have established that one of possible spots for successful development of biological pollution is the water delivery channel between the accumulating reservoir for drinking water and the device for water dispensing to the consumer since this channel as a rule is of a small cross-section area and complicated shape which makes difficult its regular cleaning without additional equipment, and also as it is located in temperature conditions most favourable for development of biological pollution.

Likewise, if regular treatment of water dispensers allows properly processing of easy-accessible areas, in particular reservoirs for drinking water, then the pipelines are difficult to be treated since as a rule they are located in hardly-accessible places, have small cross-section areas and therefore special means are required for their cleaning. Besides, some water usually remains in pipelines and when dispensers are stored in warehouses or during their transportation such places serve for most active proliferation of biological pollution and formation of biofilm. Therefore the need exists for devices preventing the accumulation of pollution in hard-to-reach places.

### Summary of Invention

Above-mentioned objectives, jointly or separately, are achieved by that the proposed disinfection device for water dispenser, which is intended for installation into a water dispenser having an accumulating reservoir for drinking water filled from an external source of drinking water, a device for dispensing water to a consumer, and a delivery pipeline for water delivery from the accumulating reservoir for drinking water to the water dispensing device, contains:
- a flow-through ozone-air mixture generator provided with an air pump for delivery of external air via an air filter to the ozone-air mixture generator, where an output of the ozone-air mixture generator is connected with the delivery pipeline for delivery of ozone-air mixture or air inside the delivery pipeline;
- a non-return valve mounted between the delivery pipeline and the ozone-air mixture generator to prevent water from getting into the ozone-air mixture generator from the delivery pipeline;
- an electromechanical ultrasonic frequency irradiator mounted on the delivery pipeline to generate ultrasonic oscillations in the delivery pipeline walls and in water filling the delivery pipeline;
- a program control unit to control the operation of the ozone-air mixture generator, the air pump and the electromechanical irradiator for disinfection and cleaning of inside surfaces of the delivery pipeline and water therein.

Connection of the delivery pipeline with the accumulating reservoir and the water dispensing device would be desirably made via damping sealing joints preventing ultrasonic oscillation from transfer to other components of the dispenser.

The disinfection device may be additionally provided with a disinfection performance indicator and a button for one-time startup of cleaning and disinfection procedure.

In one of the embodiments the device is equipped with a contactless sensor of human presence in front of the water dispenser in order to interrupt the disinfection process upon human presence near the water dispenser.

In a preferable embodiment the device has a container for collection of spilled water and an electromechanical water dispensing device connected with the program control unit, in order to drain water contaminated during the treatment process from the delivery pipeline into the container for collection of spilled water.

In such embodiment of water dispenser disinfection device the possibility is reached of automatic self-cleaning and disinfecting the most hardly accessible parts of water dispenser, and namely, the delivery pipeline from the accumulating reservoir of drinking water to the device for water dispensing into the consumer's container.

In another embodiment the device is intended for installation into a bottle-type water dispenser where a bottle with drinking water is placed with its neck downwards onto a cone with a water-intake finger positioned in central part of the cone to open the bottle and deliver water from the bottle into a water accumulation reservoir, such device being additionally provided with an ozone-air mixture distributor mounted in the water accumulation reservoir to prevent bubbles of ozone-air mixture from getting into the bottleneck.

The program control unit may be provided with a real-time clock to activate the disinfection procedure at a preset time of day.

The delivery pipeline would be desirably mounted with a horizontal slope towards the water dispensing device not less than three degrees.

A method is also proposed for disinfection of a water dispenser having an accumulating reservoir for drinking water filled from an external source of drinking water, a water dispensing device for dispensing water to a consumer, and a delivery pipeline for water delivery from the accumulating reservoir for drinking water in to the water dispensing device, comprising the following steps:
a) providing the dispenser with a disinfection device containing a flow-through ozone-air mixture generator provided with an air pump for delivery of external air via an air filter to the ozone-air mixture generator, wherein an outlet of the ozone-air mixture generator is connected with the delivery pipeline for delivery of ozone-air mixture or air inside the delivery pipeline; a non-return valve mounted between the delivery pipeline and the ozone-air mixture generator to prevent water from getting into the ozone-air mixture generator from the delivery pipeline; an electromechanical ultrasonic frequency irradiator mounted on the delivery pipeline to generate ultrasonic oscillations in the delivery pipeline walls and in water filling the delivery pipeline; a program control unit to control the operation of the ozone-air mixture generator, the air pump and the electromechanical irradiator for disinfection and cleaning of inside surfaces of the delivery pipeline and water therein;
b) delivering ozone-air mixture into the delivery pipeline by switching on the ozone-air mixture generator and the air pump;
c) generating ultrasonic oscillations in the delivery pipeline by switching on the electromechanical ultrasonic frequency irradiator;
d) treating of the delivery pipeline by simultaneous effect of the ozone-air mixture and ultrasonic oscillations during a preset time.

Joint use of ultrasonically induced cavitation and ozone-air mixture allows most completely cleaning and disinfecting the above-mentioned pipeline. Availability of the program control unit enables performing the self-cleaning and disinfection in automatic mode without human involvement which is especially important upon lasting idle time of water dispensers in absence of service personnel.

The method may additionally include the step of termination of ozone-air mixture delivery and delivery of air upon completion of pipeline treatment.

The dispenser is advantageously provided with a container for collection of spilled water, therefore the method includes the step of draining a water portion from the delivery pipeline into the container for collection of spilled water, to be taken after steps c) or d).

In a preferable embodiment the portion of drainable water shall be from one to two inner volumes of the pipeline.

The dispenser may be additionally provided with a contactless sensor of human presence in front of the water dispenser while the method includes the step of controlling the human presence near the dispenser and the step of terminating the delivery pipeline treatment process upon detection of human presence in the dispenser's vicinity.

The method may be possibly realized at a preset time as well as upon switching on the dispenser and/or upon pressing the one-time startup button.

We have found that regular cleaning and disinfection of water channel by means of proposed device allows rarer total cleanup of dispenser as well as essentially facilitates it.

Other advantages of the proposed deice will be evident from the detailed description of embodiments.

### Definitions of used terms

Disinfection in the context of the present application is understood as reduction in the level of microbiological ambience development.

Biofilm - a colony of microbiological subjects developing immediately on surface of objects.

Ultrasonic sound for purposes of the present application is understood as elastic sound oscillations with frequency above 20 kHz.

Cavitation is understood as a formation of cavities (cavitation bubbles, or caverns) in a liquid, which are filled with vapour. Cavitation occurs as a result of local pressure reduction in liquid which may take place upon passage of high-intensity acoustic wave during the rarefaction semi-period. Extreme conditions occur during cavitation: cavity, moving along with the flow into a higher pressure area (or during the cavity compression semi-period), is locked with simultaneous irradiation of a shock wave. Cavities appear mainly on uniformities, such as fungus spores, bacteria, colonies of microorganisms (biofilm) serving as a kind of target. Under effect of ultrasonic cavitation the microorganism shells are destroyed, and then the colonies of microorganisms (biofilm) are destroyed as well.

### List of drawings

Figure 1 - general diagram of embodiment of water dispenser disinfection device;
Figure 2 - enlarged view of delivery pipeline;
Figure 3 - another embodiment of the device;
Figure 4 - yet another embodiment of the device;
Figure 5 - additional embodiment of the device;
Figure 6 - another additional embodiment of the device.

### Description of embodiments

In one embodiment the proposed disinfection device for water dispenser provides cleaning and disinfection of the delivery pipeline for water delivery from the accumulating reservoir for drinking water storage to the device for water dispensing into the consumer's container. Treatment of the pipeline is carried out by complex method with the use of mechanical effect of cavitation induced by ultrasonic waves and chemical effect of ozone from the ozone-air mixture.

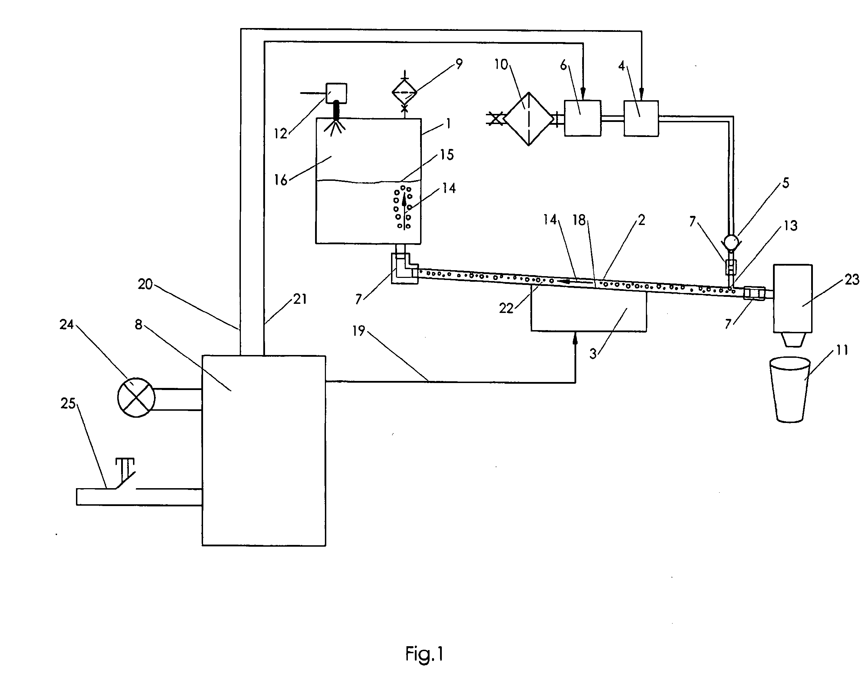

As shown in Figure 1, a disinfection device for water dispenser consists of:
- accumulating reservoir 1 for drinking water intended to store a reserve of drinking water that may be of room temperature, cooled or heated;
- delivery pipeline 2 for water delivery from accumulating reservoir 1 for drinking water into water dispensing device 23, controlled by mechanical human action, into consumer's container 11;
- electromechanical ultrasonic frequency irradiator 3 intended to obtain cavitation pockets 22 in delivery pipeline 2;
- through-flow ozone-air mixture generator 4 intended to obtain disinfecting ozone-air mixture from atmospheric air;
- non-return valve 5 mounted at inlet of ozone-air mixture generator 4, intended to prevent water from getting into ozone-air mixture generator 4;
- air pump 6 intended to deliver atmospheric air of excessive pressure into device;
- inlet atmospheric air filter 10 intended for mechanical cleaning of air coming into device;
- damping sealing joints 7 for connection of device parts exposed to effect of ultrasonic waves, in particular, delivery pipeline 2 and accumulating reservoir 1 for drinking water, delivery pipeline 2 and water dispensing device 23 controlled by mechanical human action, ozone-air mixture diffuser 13 and ozone-air mixture generator 4, intended to damp ultrasonic oscillations thus providing a longer service life of water dispenser;
- program control unit 8 of disinfection device for water dispenser, intended to control matched operation of individual parts of device and dispenser;
- ozone filter-destructor 9 intended to remove ozone from ozone-air mixture prior to discharge into atmosphere, besides, this filter-destructor serves to maintain pressure in accumulating reservoir 1 for drinking water and to clean air coming into reservoir 1 during operation of water dispenser;
- the ozone-air mixture diffuser 13 intended to deliver disinfecting ozone-air mixture into treatable pipeline 2; and
- device 12 for water dispensing into accumulating reservoir 1 for drinking water, intended to deliver drinking water into accumulating reservoir 1 and maintain preset water level 15 in reservoir 1. In this embodiment device 12 delivers water from drinking water pipeline.

The operation of the disinfection device for a water dispenser is described with reference to basic operational diagram shown in Figure 1.

When it is necessary to perform cleaning and disinfection from the formed biofilm of inside surface 18 of delivery pipeline 2 for water delivery from accumulating reservoir 1 for drinking water into water dispensing device 23 controlled by mechanical human action, program control unit 8 switches on electromechanical ultrasonic frequency irradiator 3, through-flow ozone-air mixture generator 4 and air pump 6 by means of control signals 19-21. After electromechanical ultrasonic frequency irradiator 3 is switched on, cavitation pockets 22 are formed on inner surface 18 of and inside delivery pipeline 2 and destroy biofilm on inner surface of delivery pipeline 2 and biological subjects in water inside delivery pipeline 2, at the same time via ozone-air mixture diffuser 13 there comes the ozone-air mixture formed in through-flow ozone-air mixture generator 4 from atmospheric air delivered by air pump 6 and cleaned by inlet filter 10. Simultaneous effect of ultrasonic oscillation and ozone-air mixture boosts mutual disinfecting action, biological pollution is affected both mechanically and chemically at the same time thus allowing to considerably decrease time for cleaning and disinfection of water dispenser. Ozone-air mixture flow 14 from delivery pipeline 2 carries treated water into accumulating reservoir 1 for drinking water and mix it with water containing in accumulating reservoir 1 while drinking water is enriched with ozone which additionally improves its quality and prevents biological pollution of water. Necessary ozone concentration in drinking water of accumulating reservoir 1 is provided due to various operation duration of electromechanical ultrasonic frequency irradiator 3, through-flow ozone-air mixture generator 4, and air pump 6. Decontaminated pollution residues from delivery pipeline 2 are uniformly distributed throughout water volume in accumulating reservoir 1 and do not form large local clumps. After primary cleaning residues of ozone-air mixture get into air cavity 16 of accumulating reservoir 1 for drinking water and this cavity is disinfected which prevents contamination of drinking water and formation of biofilm. Excessive pressure of ozone-air mixture is discharged into atmosphere via filter-destructor 9 wherein ozone is decomposed to oxygen and due to that poisonous cloud of ozone-air mixture is not formed in the vicinity of water dispenser. When disinfection procedure for water delivery pipeline 2 is completed, program control unit 8 disconnects electromechanical ultrasonic frequency irradiator 3 and through-flow ozone-air mixture generator 4 while air pump 6 continues working due to which excessive ozone that may deteriorate water drinking qualities is removed from water. After air flushing disinfection cycle is completed.

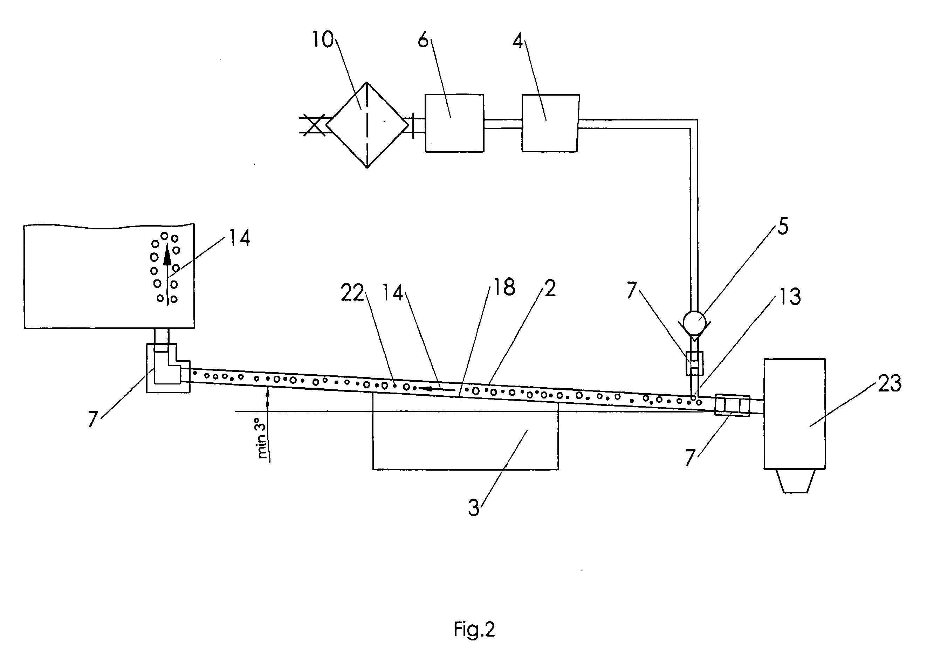

To increase formation efficiency of cavitation pockets 22 in delivery pipeline 2, this pipeline must be made of material with good conductivity of ultrasonic waves; besides, to increase operation efficiency of disinfection device for water dispenser, electromechanical ultrasonic frequency irradiator 3 must be rigidly secured on said pipeline 2; meeting these conditions allows to obtain cavitation pockets 22 throughout volume of delivery pipeline 2 for water delivery from accumulating reservoir 1 for drinking water into water dispensing device 22 and to perform treatment to the fullest extent and within shorter time. Figure 2 presents enlarged view of delivery pipeline 2 with more detailed explanation of operation of disinfection device for water dispenser. To prevent formation of air blocks in delivery pipeline 2, this pipeline, as practice shows, should be mounted with not less than 3 degrees slope in relation to water dispensing device 23, which mounting of delivery pipeline 2 provides good water circulation in pipeline and evacuation of ozone-air mixture into accumulating reservoir 1. To prevent failure of accumulating reservoir 1, water dispensing device 23, and through-flow ozone-air mixture generator 4 under effect of ultrasonic oscillations, connection of these devices with delivery pipeline 2 must be carried out via damping sealing joints 7 that, apart from leak-tight connection, provide damping of ultrasonic waves thereby protecting said devices from destruction. To protect through-flow ozone-air mixture generator 4 from water getting therein, non-return valve 5 is installed at its outlet. Program control unit 8 provides switching on/off electromechanical ultrasonic frequency irradiator 3, through-flow ozone-air mixture generator 4 and air pump 6 in accordance with a preset program, and provides signalization about performed treatment by indicator of disinfection of water dispenser 24 and upon such indication use of water dispenser is not recommended. Besides, this control unit 8 provides automatic treatment of water dispenser with preset periodicity chosen by user. As evidenced by practice, in case of water dispenser being without use for long time, minimal necessary periodicity of treatment of water dispenser must be once in 24 hours. Off-scheduled cleaning of water dispenser is provided to be carried out manually by means of one-time startup button 25. Besides, disinfection cycle of water dispenser is activated each time water dispenser is switched on thereby providing obligatory cleaning after idle time in switched-off condition.

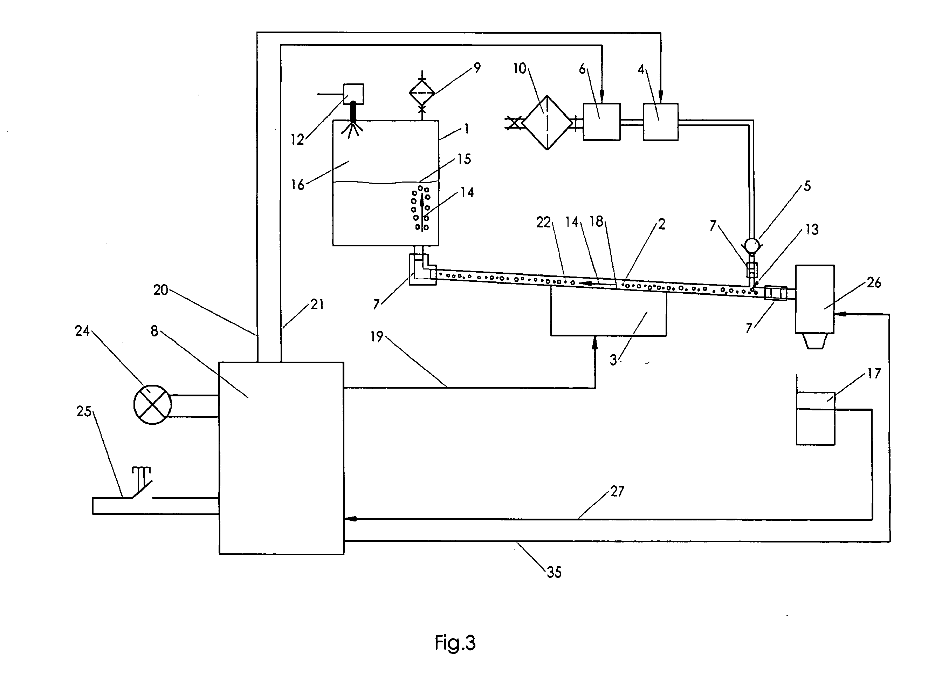

A basic operation diagram of another embodiment of the disinfection device for a water dispenser is shown in Figure 3.

This embodiment of the disinfection device for a water dispenser differs from the described one in that this device uses electromechanical water dispensing device 26 that can be controlled without human involvement and container 17 for collection of spilled water with an overfill sensor allowing to monitor how this container 17 is filled by hardware facilities. Utilization of above-mentioned distinctions makes possible realizing other cycle of self-cleaning and disinfection of pipeline 2 for water delivery from accumulating reservoir 1 of drinking water into water dispensing device 26.

When it is necessary to perform cleaning and disinfection from the formed biofilm of inside surface 18 of delivery pipeline 2 for water delivery from accumulating reservoir 1 for drinking water into electromechanical water dispensing device 26, program control unit 8 switches on electromechanical ultrasonic frequency irradiator 3. After electromechanical ultrasonic frequency irradiator 3 is switched on, cavitation pockets 22 are formed on inner surface 18 of and inside delivery pipeline 2 and destroy biofilm on inner surface of delivery pipeline 2 and biological subjects in water inside delivery pipeline 2 while air pump 6 is not switched on and ozone-air mixture is not delivered which is necessary in order cleaned mud would not get into accumulating reservoir 1; after ultrasonic cleanup procedure is completed, program control unit 8, by means of signal 35 for switching electromechanical water dispensing device 26, switches on electromechanical water dispensing device 26 for a short time in order to drain water with pollutions into container 17 for collection of spilled water with overfill sensor; whereafter through-flow ozone-air mixture generator 4 is switched on as well as air pump 6 and since that time chemical disinfection is carried out; after water saturation with ozone second water drain is carried out into container 17 for collection of spilled water whereby partial disinfection is made of an outlet channel of water dispensing device 26, the whole further disinfection cycle of water dispenser repeats the one described in the previous embodiment. In case if container 17 for collection of spilled water with overfill sensor is overfilled with water which is warned by control signal 27 - overfilled container for collection of spilled water, then water drain from water dispenser is excluded from water dispenser disinfection cycle.

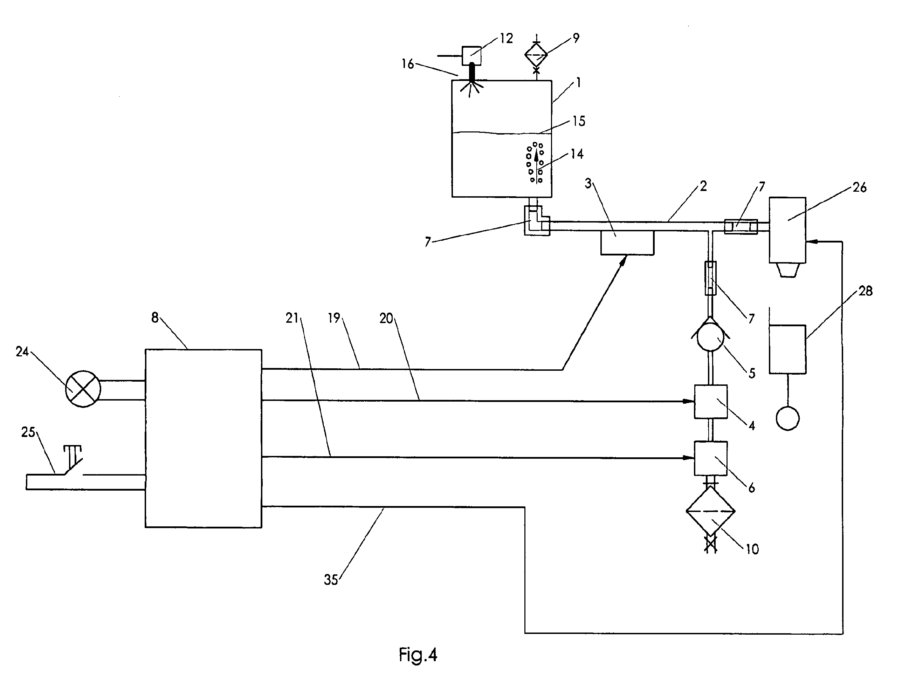

A basic operation diagram of yet another embodiment of disinfection device for water dispenser is shown in Figure 4.

This embodiment of the disinfection device for a water dispenser differs from the one described in accordance with diagram shown in Figure 3 in that this device uses container 28 for collection of spilled water with connection to external sewerage. This embodiment at all times allows draining polluted water from water dispenser and thereby cleaning and disinfecting most efficiently.

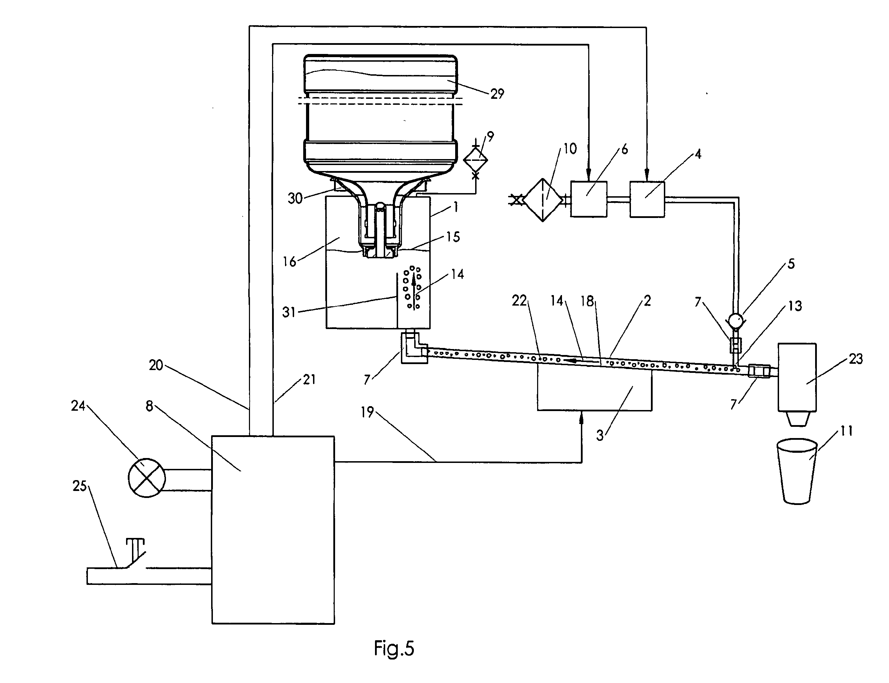

A basic operation diagram of additional embodiment of the disinfection device for a water dispenser is shown in Figure 5.

This embodiment of the disinfection device for a water dispenser differs from those described above in that this device as a source of drinking water uses bottle 29 with drinking water, cone 30 for holding the bottle its neck downwards, with a water-intake finger located in the central part of cone 30, for opening bottle 29 and water delivery from bottle 29 into reservoir 1 for water and ozone-air mixture distributer 31. When bottle 29 with drinking water is used as a source of drinking water in an embodiment of the disinfection device for a water dispenser, it should be supplemented with ozone-air mixture distributer 31 located in accumulating reservoir 1 for drinking water and intended to prevent uncontrolled getting of ozone-air mixture or air into bottle 29 with drinking water which could result in overfill of accumulating reservoir 1 and water spillage. In other respects the operation of the disinfecting device is similar to the embodiments described above.

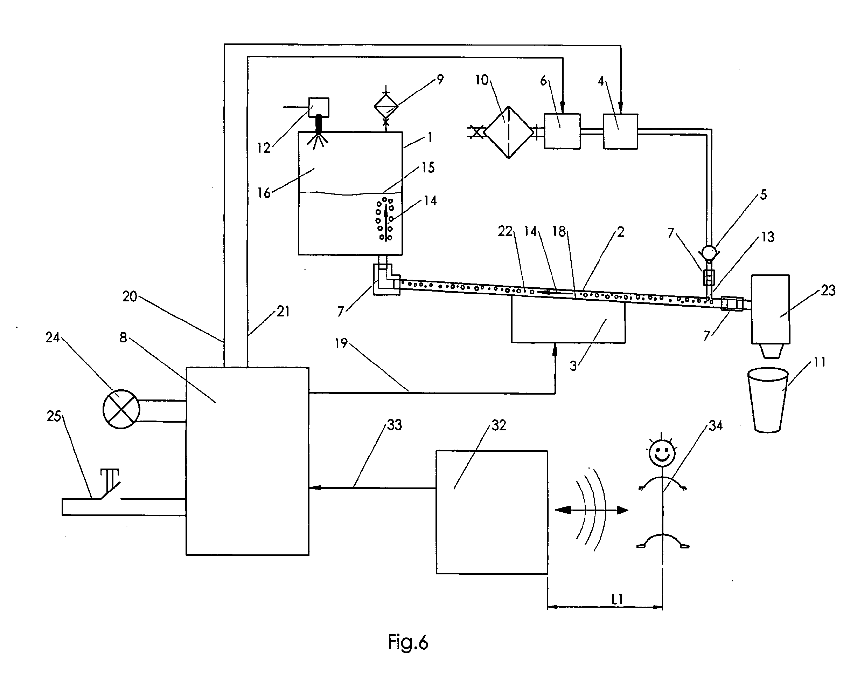

A basic operation diagram of yet another embodiment of the disinfection device for a water dispenser is presented in Figure 6.

This embodiment of the disinfection device for a water dispenser differs from those described above in that this device includes contactless sensor 32 for detecting human presence in front of the water dispenser. When control signal 33 of human presence in front of the water dispenser is received in case of person 34 being before water dispenser, program control unit 8 disconnects electromechanical ultrasonic frequency irradiator 3, through-flow ozone-air mixture generator 4, and air pump 6 to prevent effect of ultrasonic oscillation and ozone upon person 34 when taking drinking water from the water dispenser. Practice of using water dispensers shows that when taking water from a water dispenser person 34 is at distance L1 up to 500 mm, therefore contactless sensor 32 of human presence in front of the water dispenser should be adjusted for such distance to prevent unnecessary interruptions of disinfection process in the water dispenser.

### List of items in drawings

- 1: accumulating reservoir for drinking water
- 2: delivery pipeline for water delivery from drinking water accumulating reservoir into water dispensing device
- 3: electromechanical ultrasonic frequency irradiator
- 4: through-flow ozone-air mixture generator
- 5: non-return valve
- 6: air pump
- 7: damping sealing joint
- 8: program control unit
- 9: ozone filter-destructor
- 10: inlet atmospheric air filter
- 11: container for water taking by consumer
- 12: device for water delivery into accumulating reservoir
- 13: ozone-air mixture diffuser
- 14: ozone-air mixture flow
- 15: water level in accumulating reservoir for drinking water
- 16: air cavity in accumulating reservoir for drinking water
- 17: container for collection of spilled water with overfill sensor
- 18: inner surface of delivery pipeline
- 19: control signal for switching ultrasonic irradiator
- 20: control signal for switching ozone-air mixture generator
- 21: control signal for switching air pump
- 22: cavitation pockets
- 23: water dispensing device controlled by mechanical human action
- 24: indicator of water dispenser disinfection
- 25: one-time startup button
- 26: electromechanical water dispensing device
- 27: control signal - overfilled container for collection of spilled water
- 28: container for collection of spilled water with connection to external sewerage
- 29: bottle with drinking water
- 30: cone for holding bottle neck downwards with water-intake finger located in central part of cone, for opening bottle and water delivery from bottle into water reservoir inside dispenser
- 31: ozone-air mixture distributer
- 32: device of contactless detection of human presence in front of water dispenser
- 33: control signal of human presence in front of water dispenser
- 34: person
- 35: signal for switching electromechanical water dispensing device

### Citation list

US 6085540 A (DAVIS, KENNETH A) 11/07/00
US 5683576 A (HEW LYN INC) 04/11/97
US 2003000966 A (SHELTON JAMES J, ; S.I.P. TECHNOLOGIES, L.L.C) 02/01/03

## Claims

1. A disinfection device for water dispenser intended for installation into a water dispenser having
- an accumulating reservoir (1) for drinking water filled from an external source of drinking water;
- water dispensing device (23, 26) for dispensing water to a consumer; and
- a delivery pipeline (2) for water delivery from the accumulating reservoir (1) for drinking water to the water dispensing device (23, 26);
the disinfection device comprises:
- a flow-through ozone-air mixture generator (4) provided with an air pump (6) for external air delivery via an air filter (10) to the ozone-air mixture generator (4), wherein an output of the ozone-air mixture generator (4) is connected with the delivery pipeline (2) for delivery of ozone-air mixture or air inside the delivery pipeline (2);
- a non-return valve (5) mounted between the delivery pipeline (2) and the ozone-air mixture generator (4) to prevent water from getting into the ozone-air mixture generator (4) from the delivery pipeline (2);
- an electromechanical ultrasonic frequency irradiator (3) mounted on the delivery pipeline (2) to generate ultrasonic oscillations in the delivery pipeline (2) walls and in water filling the delivery pipeline (2);
- a program control unit (8) to control operation of the ozone-air mixture generator (4), the air pump (6) and the electromechanical irradiator (3) for disinfection and cleaning of inner surfaces of the delivery pipeline (2) and water therein.

2. The device of claim 1 wherein a connection of the delivery pipeline (2) with the accumulating reservoir (1) and the water dispensing device (23, 26) is made via damping sealing joints (7) preventing transfer of ultrasonic oscillations from the delivery pipeline (2) to other components of dispenser.

3. The device of claim 1 provided with an disinfection indicator (24).

4. The device of claim 1 provided with a button (25) for one-time startup of the disinfection process.

5. The device of claim 1 provided with a device (32) of contactless detection of human presence in front of the water dispenser to interrupt the disinfection process upon human presence near the water dispenser.

6. The device of claim 1, comprising a container (17, 28) for collection of spilled water and wherein the water dispensing device (26) is an electromechanical water dispensing device (26) connected with the program control unit (8) to drain water contaminated during the treatment process from the delivery pipeline (2) into the container (17, 28) for collection of spilled water.

7. The device of claim 1 intended for installation into a bottle-type water dispenser wherein a bottle (29) with drinking water is placed with its neck downwards onto a cone (30) with a water-intake finger positioned in the central part of the cone to open the bottle (29) and deliver water from the bottle (29) into the water accumulation reservoir (1), the device being additionally provided with an ozone-air mixture distributor (31) mounted in the water accumulation reservoir (1) to prevent bubbles of ozone-air mixture from getting into the neck of the bottle (29).

8. The device of claim 1 wherein the program control unit (8) includes a real-time clock to activate the disinfection procedure at a preset time of day.

9. The device of claim 1 wherein the delivery pipeline (2) is mounted with a horizontal slope towards the water dispensing device (23, 26) not less than three degrees.

10. A method for disinfection of a water dispenser having an accumulating reservoir (1) for drinking water, filled from an external source of drinking water, a water dispensing device (23, 26) for dispensing water to a consumer, and a delivery pipeline (2) for water delivery from the accumulating reservoir (1) for drinking water into the water dispensing device (23, 26), the method comprising the following steps:
a) providing the dispenser with a disinfection device containing a flow-through ozone-air mixture generator (4) and an air pump (6) for external air delivery via an air filter (10) to the ozone-air mixture generator (4) wherein an output of the ozone-air mixture generator is connected with the delivery pipeline (2) for delivery of ozone-air mixture or air inside the delivery pipeline (2); a non-return valve (5) mounted between the delivery pipeline (2) and the ozone-air mixture generator (4) to prevent water from getting into the ozone generator (4) from the delivery pipeline (2); an electromechanical ultrasonic frequency irradiator (3) mounted on the delivery pipeline (2) to generate ultrasonic oscillations in the delivery pipeline (2) walls and in water filling the delivery pipeline (2); a program control unit (8) to control operation of the ozone-air mixture generator (4), the air pump (6) and the electromechanical irradiator (3) for disinfection and cleaning of inner surfaces of the delivery pipeline (2) and water therein;
b) delivering ozone-air mixture into the delivery pipeline (2) by switching on the ozone-air mixture generator (4) and the air pump (6);
c) generating ultrasonic oscillations in the delivery pipeline (2) by switching on the electromechanical ultrasonic frequency irradiator (3);
d) treating the delivery pipeline (2) by simultaneous effect of ozone-air mixture and ultrasonic oscillations during a preset time.

11. The method of claim 10 comprising an additional step of terminating ozone-air mixture delivery into the delivery pipeline (2) upon completion of the pipeline (2) treatment.

12. The method of claim 10 comprising a step of providing the dispenser with a container (17, 28) for collecting of spilled water, and a further step f) of draining a portion of water from the delivery pipeline (2) into the container (17, 28) for collection of spilled water, the step f) being carried out after step c) or d).

13. The method of claim 12 wherein the portion of drainable water is from one to two inner volumes of the delivery pipeline (2).

14. The method of claim 10 containing a step of providing dispenser with a device (32) for contactless detection of human presence in front of the water dispenser, a step of controlling human presence near the dispenser and a step of terminating the delivery pipeline (2) treatment process upon detection of human presence in the dispenser's vicinity.

15. The method of claim 10 being realized upon switching on of the dispenser.

## Patentansprüche

1. Desinfektionsvorrichtung für Wasserspender, die zur Installation in einen Wasserspender vorgesehen ist, der Folgendes aufweist:
- ein Sammelreservoir (1) für Trinkwasser, das aus einer externen Trinkwasserquelle gefüllt wird,
- eine Wasserspendervorrichtung (23, 26) zum Abgeben von Wasser an einen Verbraucher und
- eine Zufuhrleitung (2) für die Wasserzufuhr vom Sammelreservoir (1) für Trinkwasser zur Wasserspendervorrichtung (23, 26),
wobei die Desinfektionsvorrichtung Folgendes umfasst:
- einen Durchlaufgenerator für eine Ozon-Luft-Mischung (4), der mit einer Luftpumpe (6) zur externen Luftzufuhr über einen Luftfilter (10) zu dem Generator für die Ozon-Luft-Mischung (4) ausgestattet ist, wobei ein Ausgang des Generators für die Ozon-Luft-Mischung (4) mit der Zufuhrleitung (2) verbunden ist, um Ozon-Luft-Mischung oder Luft im Inneren der Zufuhrleitung (2) zuzuführen,
- eine Rückflusssperre (5), die zwischen der Zufuhrleitung (2) und dem Generator für die Ozon-Luft-Mischung (4) montiert ist, um zu verhindern, dass Wasser aus der Zufuhrleitung (2) in den Generator für die Ozon-Luft-Mischung (4) gelangt,
- ein elektromechanisches Ultraschallfrequenz-Bestrahlungsgerät (3), das an der Zufuhrleitung (2) montiert ist, um Ultraschallschwingungen in den Wänden der Zufuhrleitung (2) und in Wasser, das die Zufuhrleitung (2) füllt, zu erzeugen,
- eine Programmsteuereinheit (8) zum Steuern des Betriebs des Generators für die Ozon-Luft-Mischung (4), der Luftpumpe (6) und des elektromechanischen Bestrahlungsgerätes (3) zum Desinfizieren und Reinigen der Innenflächen der Zufuhrleitung (2) und des Wassers darin.

2. Vorrichtung nach Anspruch 1, wobei eine Verbindung der Zufuhrleitung (2) mit dem Sammelreservoir (1) und der Wasserspendervorrichtung (23, 26) über dämpfende Dichtungsverbindungsstücke (7) hergestellt wird, die die Übertragung von Ultraschallschwingungen von der Zufuhrleitung (2) auf andere Komponenten des Spenders verhindert.

3. Vorrichtung nach Anspruch 1, die mit einem Desinfektionsindikator (24) ausgestattet ist.

4. Vorrichtung nach Anspruch 1, die mit einer Taste (25) zum einmaligen Starten des Desinfektionsprozesses ausgestattet ist.

5. Vorrichtung nach Anspruch 1, die mit einer Vorrichtung (32) zur berührungsfreien Erkennung einer menschlichen Präsenz vor dem Wasserspender ausgestattet ist, um den Desinfektionsprozess bei menschlicher Präsenz in der Nähe des Wasserspenders zu unterbrechen.

6. Vorrichtung nach Anspruch 1, die einen Behälter (17, 28) zum Auffangen verschütteten Wassers umfasst und wobei die Wasserspendervorrichtung (26) eine elektromechanische Wasserspendervorrichtung (26) ist, die mit der Programmsteuereinheit (8) verbunden ist, um Wasser, das während des Behandlungsprozesses verunreinigt wird, aus der Zufuhrleitung (2) in den Behälter (17, 28) zum Auffangen verschütteten Wassers abzuführen.

7. Vorrichtung nach Anspruch 1, die zur Installation in einen Flaschenwasserspender vorgesehen ist, wobei eine Flasche (29) mit Trinkwasser mit dem Hals nach unten auf einen Kegel (30) mit einem Wassereinlauffinger, der im mittigen Teil des Kegels positioniert wird, gesetzt wird, um die Flasche (29) zu öffnen und Wasser von der Flasche (29) in das Wassersammelreservoir (1) zuzuführen, wobei die Vorrichtung außerdem mit einem in dem Wassersammelreservoir (1) montierten Verteiler für die Ozon-Luft-Mischung (31) ausgestattet ist, um zu verhindern, dass Blasen der Ozon-Luft-Mischung zurück in den Hals der Flasche (29) gelangen.

8. Vorrichtung nach Anspruch 1, wobei die Programmsteuereinheit (8) eine Echtzeituhr beinhaltet, um das Desinfektionsverfahren zu einer voreingestellten Tageszeit zu beginnen.

9. Vorrichtung nach Anspruch 1, wobei die Zufuhrleitung (2) mit einer horizontalen Neigung zur Wasserspendervorrichtung (23, 26) hin von nicht weniger als drei Grad montiert ist.

10. Verfahren zum Desinfizieren eines Wasserspenders, der ein Sammelreservoir (1) für Trinkwasser, das aus einer externen Trinkwasserquelle gefüllt wird, eine Wasserspendervorrichtung (23, 26) zum Abgeben von Wasser an einen Verbraucher und eine Zufuhrleitung (2) für die Wasserzufuhr vom Sammelreservoir (1) für Trinkwasser zur Wasserspendervorrichtung (23, 26) aufweist, wobei das Verfahren die folgenden Schritte umfasst:
a) Ausstatten des Spenders mit einer Desinfektionsvorrichtung, die einen Durchlaufgenerator für eine Ozon-Luft-Mischung (4) und eine Luftpumpe (6) zur externen Luftzufuhr über einen Luftfilter (10) zu dem Generator für die Ozon-Luft-Mischung (4) enthält, wobei ein Ausgang des Generators für die Ozon-Luft-Mischung mit der Zufuhrleitung (2) verbunden ist, um Ozon-Luft-Mischung oder Luft im Inneren der Zufuhrleitung (2) zuzuführen, mit einer Rückflusssperre (5), die zwischen der Zufuhrleitung (2) und dem Generator für die Ozon-Luft-Mischung (4) montiert ist, um zu verhindern, dass Wasser aus der Zufuhrleitung (2) in den Ozon-Generator (4) gelangt, mit einem elektromechanischen Ultraschallfrequenz-Bestrahlungsgerät (3), das an der Zufuhrleitung (2) montiert ist, um Ultraschallschwingungen in den Wänden der Zufuhrleitung (2) und in Wasser, das die Zufuhrleitung (2) füllt, zu erzeugen, mit einer Programmsteuereinheit (8) zum Steuern des Betriebs des Generators für die Ozon-Luft-Mischung (4), der Luftpumpe (6) und des elektromechanischen Bestrahlungsgerätes (3) zum Desinfizieren und Reinigen der Innenflächen der Zufuhrleitung (2) und des Wassers darin,
b) Zuführen von Ozon-Luft-Mischung in die Zufuhrleitung (2) durch Anschalten des Generators für die Ozon-Luft-Mischung (4) und der Luftpumpe (6),
c) Erzeugen von Ultraschallschwingungen in der Zufuhrleitung (2) durch Anschalten des elektromechanischen Ultraschallfrequenz-Bestrahlungsgerätes (3),
d) Behandeln der Zufuhrleitung (2) durch die gleichzeitige Wirkung von Ozon-Luft-Mischung und Ultraschallschwingungen während einer voreingestellten Zeit.

11. Verfahren nach Anspruch 10, das einen zusätzlichen Schritt des Beendens der Zufuhr von Ozon-Luft-Mischung in die Zufuhrleitung (2) nach Abschluss der Behandlung der Leitung (2) umfasst.

12. Verfahren nach Anspruch 10, das einen Schritt des Ausstattens des Spenders mit einem Behälter (17, 28) zum Auffangen verschütteten Wassers umfasst und einen weiteren Schritt f) des Abführens eines Teils des Wassers aus der Zufuhrleitung (2) in den Behälter (17, 28) zum Auffangen verschütteten Wassers, wobei der Schritt f) nach dem Schritt c) oder d) ausgeführt wird.

13. Verfahren nach Anspruch 12, wobei der Teil des abführbaren Wassers ein bis zwei Innenvolumina der Zufuhrleitung (2) beträgt.

14. Verfahren nach Anspruch 10, das einen Schritt des Ausstattens des Spenders mit einer Vorrichtung (32) zur berührungsfreien Erkennung einer menschlichen Präsenz vor dem Wasserspender, einen Schritt des Kontrollierens einer menschlichen Präsenz in der Nähe des Spenders und einen Schritt des Beendens des Behandlungsprozesses der Zufuhrleitung (2) bei Erkennen menschlicher Präsenz in der Nähe des Spenders enthält.

15. Verfahren nach Anspruch 10, das bei Anschalten des Spenders ausgeführt wird.

## Revendications

1. Un dispositif de désinfection pour distributeur d'eau, destiné à être installé dans un distributeur d'eau ayant
- un réservoir d'accumulation (1) pour eau potable rempli depuis un irradiateur externe d'eau potable ;
- un dispositif de distribution d'eau (23, 26) servant à distribuer de l'eau à un consommateur ; et
- une conduite de distribution (2) pour fourniture d'eau depuis le réservoir d'accumulation (1) pour eau potable vers le dispositif de distribution d'eau (23, 26) ;
le dispositif de désinfection comprend :
- un générateur (4) de mélange ozone-air à circulation muni d'une pompe à air (6) pour fourniture d'air extérieur via un filtre à air (10) vers le générateur de mélange ozone-air (4), dans lequel une sortie du générateur de mélange ozone-air (4) est raccordée à la conduite de distribution (2) pour fourniture d'un mélange ozone-air ou d'air à l'intérieur de la conduite de distribution (2) ;
- un clapet anti-retour (5) installé entre la conduite de distribution (2) et le générateur de mélange ozone-air (4) pour éviter que l'eau ne pénètre dans le générateur de mélange ozone-air (4) depuis la conduite de distribution (2) ;
- un irradiateur électromécanique à fréquence ultrasonore (3) installée sur la conduite de distribution (2) afin de produire des oscillations ultrasonores dans les parois de la conduite de distribution (2) ainsi que dans l'eau qui remplit la conduite de distribution (2) ;
- une unité de commande programmable (8) pour commander le fonctionnement du générateur de mélange ozone-air (4), de la pompe à air (6) et de l'irradiateur électromécanique (3) pour désinfecter et nettoyer les surfaces internes de la conduite de distribution (2) et de l'eau y contenue.

2. Le dispositif de la revendication 1 dans lequel un raccord reliant la conduite de distribution (2) au réservoir d'accumulation (1) et au dispositif de distribution d'eau (23, 26) est réalisé avec des joints d'étanchéité amortisseurs (7) qui empêchent le transfert d'oscillations ultrasonores de la conduite de distribution (2) à d'autres composants du distributeur.

3. Le dispositif de la revendication 1 équipé d'un indicateur de désinfection (24).

4. Le dispositif de la revendication 1 équipé d'un bouton (25) servant au démarrage ponctuel du processus de désinfection.

5. Le dispositif de la revendication 1 équipé d'un dispositif (32) de détection sans contact de présence humaine devant le distributeur d'eau afin d'interrompre le processus de désinfection en cas de présence humaine à proximité du distributeur d'eau.

6. Le dispositif de la revendication 1, comprenant un récipient (17, 28) de collecte d'eau de déversement et dans lequel le dispositif de distribution d'eau (26) est un dispositif de distribution d'eau électromécanique (26) connecté à l'unité de commande programmable (8) afin d'évacuer l'eau contaminée au cours du processus de traitement depuis la conduite de distribution (2) dans le récipient (17, 28) pour recueillir l'eau de déversement.

7. Le dispositif de la revendication 1 destiné à être installé dans un distributeur d'eau de type bouteille, dans lequel une bouteille (29) avec de l'eau potable est placée avec son goulot vers le bas dans un cône (30), avec un doigt d'admission d'eau situé dans la partie centrale du cône afin d'ouvrir la bouteille (29) et de distribuer de l'eau de la bouteille (29) dans le réservoir d'accumulation d'eau (1), le dispositif étant de plus équipé d'un distributeur de mélange ozone-air (31) installé dans le réservoir d'accumulation d'eau (1) pour empêcher les bulles du mélange ozone-air de pénétrer dans le goulot de la bouteille (29).

8. Le dispositif de la revendication 1 dans lequel l'unité de commande programmable (8) comporte une horloge temps réel pour activer la procédure de désinfection à une heure prédéterminée de la journée.

9. Le dispositif de la revendication 1 dans lequel la conduite de distribution (2) est installée avec une pente horizontale vers le dispositif de distribution d'eau (23, 26) d'au moins trois degrés.

10. Une méthode de désinfection d'un distributeur d'eau ayant un réservoir d'accumulation (1) pour eau potable, rempli depuis un irradiateur externe d'eau potable, un dispositif de distribution d'eau (23, 26) servant à distribuer de l'eau à un consommateur, et une conduite de distribution (2) servant à la distribution d'eau depuis le réservoir d'accumulation (1) pour eau potable dans le dispositif de distribution d'eau (23, 26), la méthode comprenant les étapes suivantes :
a) munir le distributeur d'un dispositif de désinfection contenant un générateur (4) de mélange ozone-air à circulation et une pompe à air (6) servant à la fourniture d'air extérieur via un filtre à air (10) vers le générateur de mélange ozone-air (4), dans lequel une sortie du générateur de mélange ozone-air est raccordée à la conduite de distribution (2) pour fournir un mélange ozone-air ou de l'air à l'intérieur de la conduite de distribution (2) ; un clapet anti-retour (5) monté entre la conduite de distribution (2) et le générateur de mélange ozone-air (4) afin d'éviter que de l'eau ne pénètre dans le générateur d'ozone (4) depuis la conduite de distribution (2) ; un irradiateur électromécanique à fréquence ultrasonore (3) installée sur la conduite de distribution (2) afin de produire des oscillations ultrasonores dans les parois de la conduite de distribution (2) ainsi que dans l'eau qui remplit la conduite de distribution (2) ; une unité de commande programmable (8) pour commander le fonctionnement du générateur de mélange ozone-air (4), de la pompe à air (6) et de l'irradiateur électromécanique (3) dans le but de désinfecter et nettoyer les surfaces internes de la conduite de distribution (2) et de l'eau y contenue,
b) fournir le mélange ozone-air dans la conduite de distribution (2) en mettant en marche le générateur de mélange ozone-air (4) et la pompe à air (6) ;
c) enregistrer des oscillations ultrasonores dans la conduite de distribution (2) en mettant en marche l'irradiateur électromécanique à fréquence ultrasonore (3) ;
d) traiter la conduite de distribution (2) par l'action simultanée du mélange ozone-air et des oscillations ultrasonores pendant une durée prédéterminée.

11. La méthode de la revendication 10 comprenant une étape supplémentaire de cessation de la distribution du mélange ozone-air dans la conduite de distribution (2) une fois terminé le traitement de la conduite (2).

12. La méthode de la revendication 10 comprenant une étape d'équipement du distributeur d'un récipient (17, 28) de collecte des déversements d'eau, et une étape supplémentaire f) d'évacuation d'une partie de l'eau de la conduite de distribution (2) dans le récipient (17, 28) de collecte des déversements d'eau, l'étape f) étant exécutée après l'étape c) ou d).

13. La méthode de la revendication 12 dans laquelle la partie de l'eau pouvant être évacuée est de un à deux volumes intérieurs de la conduite de distribution (2).

14. La méthode de la revendication 10 contenant une étape d'équipement du distributeur d'un dispositif (32) de détection sans contact de présence humaine devant le distributeur d'eau, une étape de contrôle de présence humaine à proximité du distributeur, et une étape de cessation du processus de traitement de la conduite de distribution (2) en cas de détection d'une présence humaine aux alentours du distributeur.

15. La méthode de la revendication 10 étant réalisée lors de la mise en marche du distributeur.
